# EUROPEAN PATENT SPECIFICATION

(11) **EP 2 099 603 B1**
(45) Date of publication and mention of the grant of the patent: **20.02.2013**
(21) Application number: 07854641.3
(22) Date of filing: 14.11.2007
(51) Int. Cl.: A61N 1/375

(54) **OPTICAL MEMBER**
OPTISCHES GLIED
ELEMENT OPTIQUE

(30) Priority: 14.11.2006 US 865763 P
(43) Date of publication of application: 16.09.2009
(73) Proprietor: MEDTRONIC, INC., Minneapolis, Minnesota 55432 (US)
(72) Inventor: THOM, Andrew, Maple Grove, Minnesota 55369 (US); TETTEMER, Susan A., Fridley, Minnesota 55421 (US); REITERER, Markus W., Plymouth, Minnesota 55422 (US)
(74) Representative: Hughes, Andrea Michelle
(86) International application number: PCT/US2007/084664
(87) International publication number: WO 2008/061135

(56) References cited:
- WO-A-01/24962
- US-A- 5 902 326

## Description

### FIELD OF THE INVENTION

The present invention generally relates to an implantable medical device (IMD) and, more specifically, to formation of an optical member associated with the IMD.

### BACKGROUND OF THE INVENTION

Implantable medical devices (IMDs) detect one or more physiological conditions and, in response to a detected condition, may then deliver therapy to a patient. Exemplary IMDs include implantable pulse generators (IPGs), implantable cardioverter-defibrillators (ICDs), myostimulators, neurological stimulators, drug delivery devices, insulin pumps, glucose monitors or other suitable devices. ICDs typically comprise, inter alia, a control module, memory, a capacitor, and a battery that are housed in a hermetically sealed container. A lead, which extends from the ICD to a patient's tissue, typically includes a physiological sensor. The physiological sensor signals the control module as to a patient's physiological condition. The control module then compares data from the sensed signal to data stored in memory.

An optical sensor, a type of physiological sensor, can detect changes in light modulation due to a change in a physiological condition (e.g. oxygen saturation levels, glucose level etc.) in body fluid or tissue. An exemplary optical sensor is described in U.S. Patent No.5,902632 issued to Lessar et al. in which the optical sensor includes a ferrule coupled to a window with a gold brazed joint. It is desirable to continue to develop optical sensors to detect data related to parameters that assists in diagnosing and/or treating a medical condition. The invention provides a method as defined in the independent claim(s).

### BRIEF DESCRIPTION OF THE DRAWINGS

FIG. 1 is a conceptual schematic view of a medical device system;
FIG. 2A depicts a schematic cross-sectional view of an optical member;
FIG. 2B depicts a schematic cross-sectional view of another optical member;
FIG. 3A is a schematic top view of an exemplary ferrule;
FIG. 3B is an schematic top angled view of an exemplary ferrule;
FIG. 3C is a schematic cross-sectional view of an exemplary ferrule;
FIG. 3D is an enlarged of a schematic cross-sectional view of strain relief of an exemplary ferrule;
FIG. 4A is a schematic top view of an exemplary clear member or window;
FIG. 4B is an outer perimeter of the exemplary clear member depicted in FIG. 4A;
Fig 4C is a schematic cross-sectional view of the exemplary clear member depicted in FIGs. 4A-4B;
FIG. 5 is a schematic cross-sectional view of the components for forming an optical member placed into a setter plate for diffusion bonding;
FIG. 6 is a schematic cross-sectional view of a multilayer assembly for forming the optical member depicted in FIG. 5; and
FIG. 7 is a flow diagram for forming an optical member.

### DETAILED DESCRIPTION

The following description of an embodiment is merely exemplary in nature and is in no way intended to limit the invention, its application, or uses. For purposes of clarity, the same reference numbers are used in the drawings to identify similar elements. As used herein, the term "module" refers to an application specific integrated circuit (ASIC), an electronic circuit, a processor (shared, dedicated, or group) and memory that execute one or more software or firmware programs, a combinational logic circuit, or other suitable components that provide the described functionality. Additionally, the term "opto-electronic device", also referred to herein as "opto-electronic component", refers to any electrical circuit component capable of emitting light in response to an applied voltage or current or emitting current in response to exposure to light, including for example light emitting diodes (LEDs), vertical cavity surface emitting lasers (VCSELs), photoresistors or light dependent resistors, photodiodes, phototransistors, photovoltaic cells, or charge-coupled devices.

Generally, the present invention is directed to formation of an optical member that is used in combination with an opto-electronic component to create an optical sensor. The optical member includes a clear member or a window diffusion bonded to a ferrule. The optical member lacks joint material between the clear member and the ferrule. The optical member is then coupled to housing and/or a lead associated with an implantable medical device (IMD).

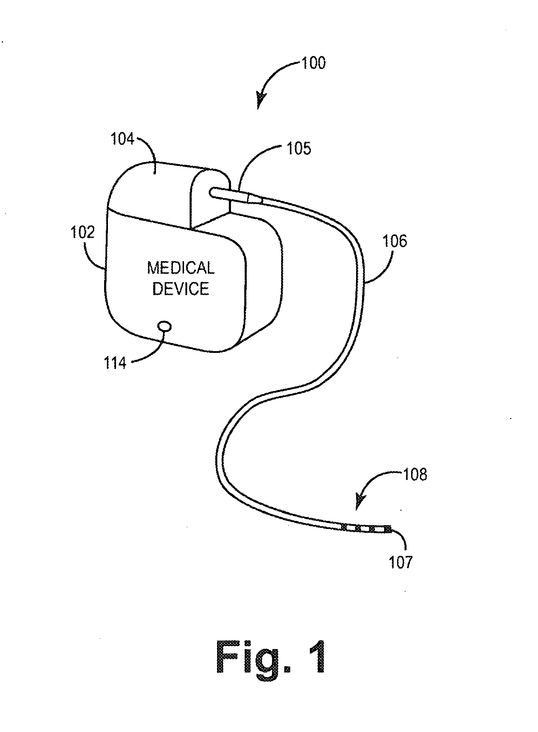

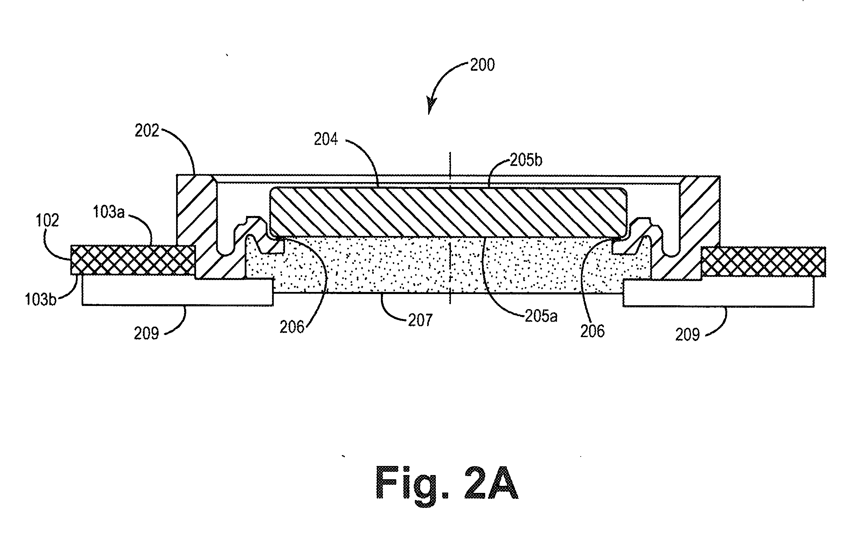

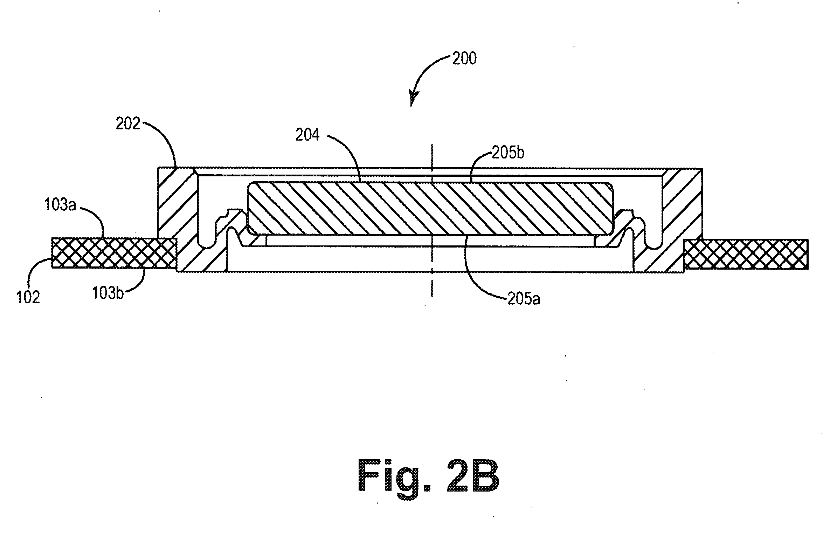

**FIG. 1** depicts a medical device system 100. A medical device system 100 includes a medical device housing 102 having a connector module 104 that electrically couples various internal electrical components of medical device housing 102 to a proximal end 105 of a medical lead 106. Referring briefly to **FIG. 2A**, housing 102 includes inner surface 103a that covers electronic components therein and an outer surface 103b exposed to the body and tissue. A medical device system 100 may comprise any of a wide variety of medical devices that include one or more medical lead(s) 106 and circuitry coupled to the medical lead(s) 106. By way of example, medical device system 100 may take the form of an implantable cardiac pacemaker that provides therapeutic stimulation to the heart or a neurostimulator. Alternatively, medical device system 100 may take the form of an implantable cardioverter, an implantable defibrillator, an implantable cardiac pacemaker-cardioverter-defibrillator (PCD), or an implantable medical device that solely monitors conditions associated with the patient. Medical device system 100 includes one or more optical sensors 114 to detect a physiological condition (e.g. oxygen in blood etc.). Exemplary implantable optical sensors used for monitoring blood oxygen saturation is generally disclosed in commonly assigned U.S. Patent No. 6,198,952 issued to Miesel and U.S. Patent No. 5,902,632 issued to Lessar et al.

**FIG 2A** depicts, by way of background only, an optical member 200 (also referred to as a window component) that is combined with an opto-electronic component (not shown) to form an optical sensor. Optical member 200 includes a ferrule 202, a clear member 204 or window, a joint 206, an optional washer 209, and an optional thin protective coating placed over clear member's 204 first surface 205a but not over second surface 205b.

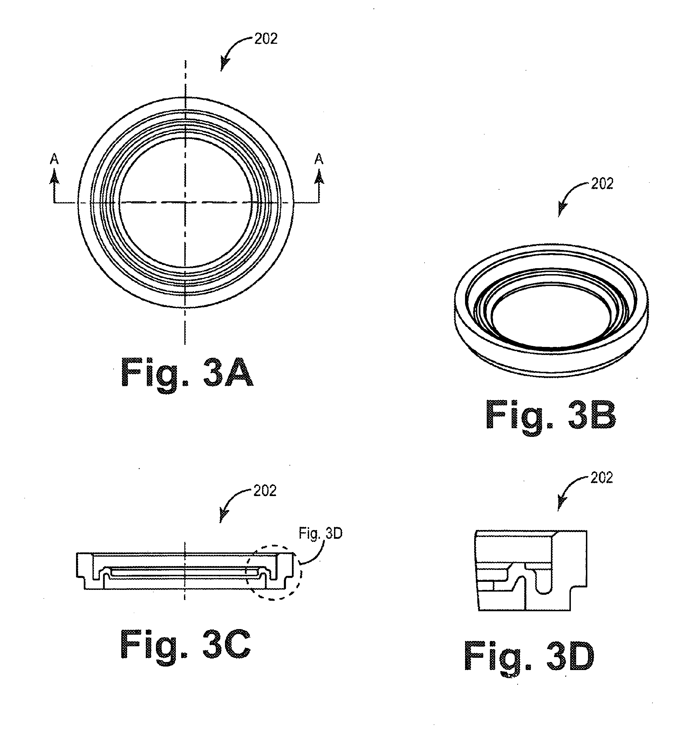

Ferrule 202, depicted in **FIGs. 2A-2B** **and 3A-3D**, given by way of background only, is described in greater detail in U.S. Patent No. 5,902,236 issued to Lessar et al. Skilled artisans appreciate that a variety of other ferrules may be used. Ferrule 202 comprises conductive material. Exemplary conductive material includes medical grade titanium typically of grade 2 or grade 4 designation, medical grade niobium, or an alloy of titanium and niobium (e.g. Ti-45Nb).

Joint material 206 may comprise a variety of joint materials except gold.

Exemplary joint material includes medical grade pure copper (Cu) (i.e. unalloyed copper such that titanium or silver are not added or alloyed with the copper) titanium copper (TICUSIL^{®} alloys (i.e. 68.5 weight% (wt%) Ag, 26.7wt%, and Cu and 4.5wt% Ti) (e.g. Ag-Cu eutectic + 4.5 wt% Ti), pure aluminum (Al), Cu-Ti alloys, Cu-Ti alloys containing any of the following ternary elements tin (Sn), indium (In), Al, silver (Ag), quaternary silver-nickel-molybdenum-vanadium (Ag-Ni-Mo-V) alloys, silver copper oxide (Ag-CuO) alloy, and/or silver-silicon Ag-Si eutectic alloys. At least one of the previous listed joint materials may be combined or alloyed with a rare earth metal (e.g. hafnium etc.) to form joint material 206.

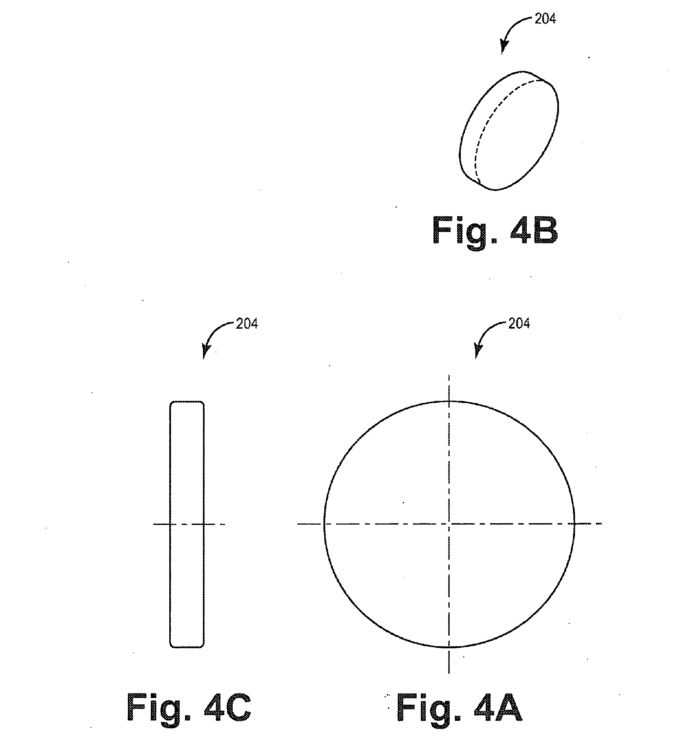

**FIGs. 4A-4C** depict an exemplary clear member 204 or window. Clear member 204 is depicted as circular in shape but may be formed in a variety of suitable shapes (e.g. square, rectangular, at least one triangular formed in the shape (i.e. triangle, hexagon etc.), or non-shapes. Additionally, clear member 204 may be formed of a variety of materials. For example, clear member 204 may comprise sapphire (e.g. aluminum oxide, single crystal aluminum oxide, etc.) Sapphire, a crystalline structure which possesses a c-axis oriented from 0 degrees to 90 degrees to the face of clear member 204, exhibits excellent transmission properties in the optical wavelengths of ultraviolet to visible to infrared wavelengths up to 5 microns.

Exemplary protective coatings may be optionally used to protect the exposed edges of joint material 206 and/or the interface between ferrule 202 and clear member 204 from long-term exposure to the body environment. Protective coatings include flexible polydimethylsiloxanes (e.g. Nusil 1137 and Nusil 2000 commercially available from Nusil Silicone Technology, Carpinteria, CA), hexane in combination with the adhesives listed above and/or rigid epoxies, (e.g. aliphatic amine cured bisphenol epoxy adhesive) .

Partial covering washer 209 is depicted adjacent to ferrule 202, housing 102, and protective coating 207. Covering washer 209 is also optionally used to protect the exposed edges of the protective coating 207 on clear member 204 from long-term exposure to the body environment. Washer 209 also prevents any potential delamination of the coating during routine handling of the medical device system 100.

Numerous optical members 200 may be formed through a diffusion bonding process. Table 1 summarizes exemplary dimensions of the components that may be used to form optical member 200.

**Table 1-Exemplary dimensions for a ferrule and a window**

| Clear member or Window diameter (inches) | Ferrule inner diameter (ID) (inches) | Ferrule outer diameter (OD) (inches) | Ferrule Thickness (inches) |
|---|---|---|---|
| 0.098 (2.49 mm) | 0.086 (2.184 mm) | 0.178 (4.521 mm) | 0.040 (1.016 mm) |
| 0.130 (3.302 mm) | 0.118 (2.997 mm) | 0.210 (5.334 mm) | 0.040 (1.016 mm) |
| 0.160 (4.064 mm) | 0.148 (3.759 mm) | 0.240 (6.096 mm) | 0.040 (1.016 mm) |
| 0.180 (4.572 mm) | 0.168 (4.267 mm) | 0.260 (6.604 mm) | 0.040 (1.016 mm) |
| 0.200 (5.08 mm) | 0.188 (4.775 mm) | 0.280 (7.112 mm) | 0.040 (1.016 mm) |

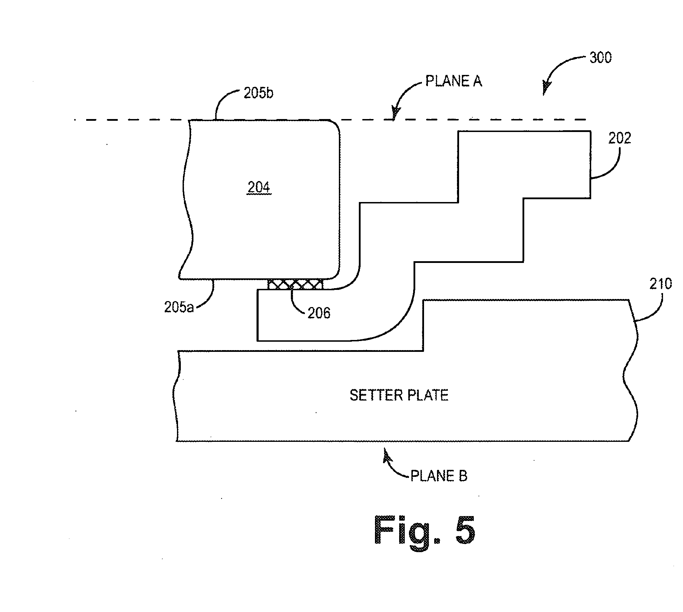

**FIGs. 5-6** depict a portion of an assembly process for an optical member 200 in which diffusion bonding is applied. In this embodiment, optical member 200 is seated in a cup cavity 208 of a setter plate 210. Setter plate 210 is suitably designed so that it remains rigid during the application of load and does not react with optical member 200 components materials. Cup cavity 208 is dimensioned to receive ferrule 202 and to maintain ferrule 202 in a controlled position. Ferrule 202 is inserted into cup cavity 208.

Prior to or after ferrule 202 is inserted into a cup cavity 208, in one embodiment, a metallization layer is introduced over the first surface 205a of clear member 204 to promote bonding between clear member 204 and ferrule 202. Metallization layer can be introduced over first surface 205a through sputtering, screen printing or other suitable methods. An exemplary metallization layer comprises one or more noble metals (e.g. platinum or palladium, silver, tantalum, or rhodium), and/or other suitable materials (e.g. Ti, Nb, rare earth metals such as hafnium etc.). Second surface 205b of clear member 204 lacks a protective coating. Clear member 204 is then placed into ferrule 202, with the metallized first surface 205a of the clear member 204 in direct contact with ferrule 202. Together, the subassembly 300 in **FIG. 5** comprises a single layer.

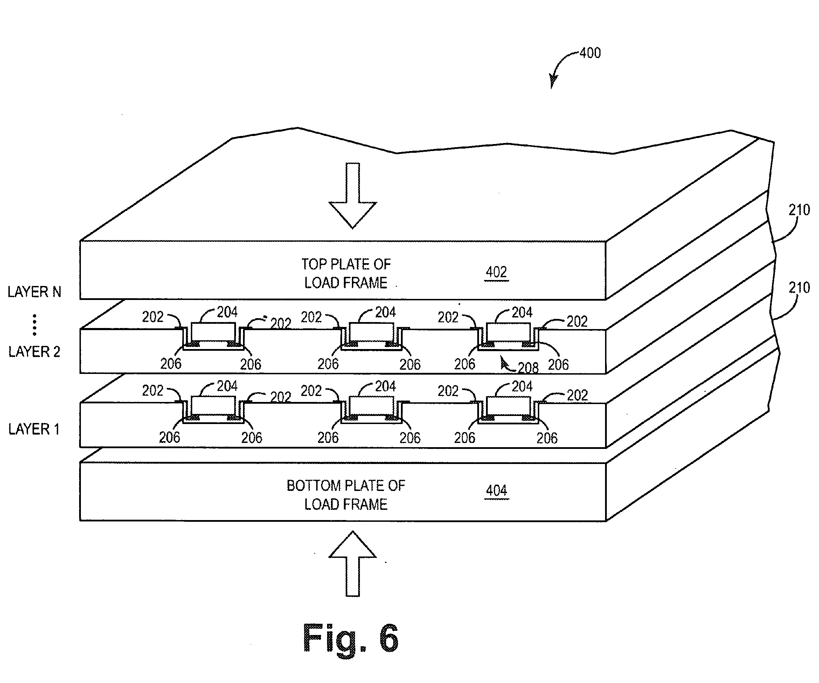

Referring to **FIG. 6****,** stacked assembly 400 depicts multiple layers, stacked one on top of each other, with the outer layers being sandwiched between a top plate 402 (also referred to as a first plate) and bottom plate 404 (also referred to as a second plate). Individual layers of setter plates 210 may contain X rows and Y columns of optical member 200. Thus, each layer contains X●Y components, and a stack ofN layers contains the product of X●Y●N optical members 200.

A top plate 402 and bottom plate 404 are assembled on the stack of N layers, and this assembled stack 400 is inserted into a vacuum hot press furnace or oven (not shown) that is capable of applying a suitable compressive load against the outer surfaces of top and bottom plates 402, 404 to achieve the desired contact pressure of about 5-25 megapascals (MPa) between the clear member 204 and ferrule 202. An exemplary oven may be a series 3250 model 6-1650-15T Diffusion Bonding Vacuum Hot Press available from Centorr Vacuum Industries, Nashua, New Hampshire. As illustrated in **FIG. 5**, the application of compressive load is localized to the contact area between the metallized portion 206 of the clear member 204 and ferrule 202 by establishing two planes of contact, denoted as plane A and plane B, for each layer. Load is transmitted from setter plate 210 to ferrule 202, and adjoining contact with the metallized clear member 204. Plane A indicates the position of the bottom of the setter plate 210 for the next adjacent layer, and thus the compression of the assembled stack of layers leads to the simultaneous loading of all optical member 200 components within the assembled stack 400.

Assembled stack 400 is placed in a vacuum hot press oven or furnace (not shown) and a compressive load is applied to achieve the desired contact pressure. The furnace is then heated in a controlled atmosphere, either in an inert atmosphere or under vacuum. An inert atmosphere can be introduction of argon into the chamber (not shown) of the oven whereas an atmosphere under vacuum may be a pressure of equal to or less than 1x10⁻⁵ Torr. Stacked assembly 400 is held in the oven as long as 12 hours at lower temperatures of 600°-800° Celsius (C), and as short as 0.5-2 hours at 1300-1400°C. The combined effect of applied pressure and elevated temperature leads to the formation of a solid state diffusion bond between the clear member 204 and ferrule 202. Upon completion of the thermal cycle, the assembled stack 400 is removed from the hot press furnace and the diffusion bonded optical member 200 components are removed from the setter plates 210. Optical member 200 components can then be joined to a suitable device housing 102 and welded into place using standard metal joining techniques. Clear member 204 can then function as an effective optical feedthrough, enabling the sensing of blood oxygenation for either tissue perfusion sensing, if mounted into an implantable medical device, or enabling measurement of mixed venous oxygen saturation (SvO₂), if mounted into a housing on an implanted lead within the heart or a blood vessel.

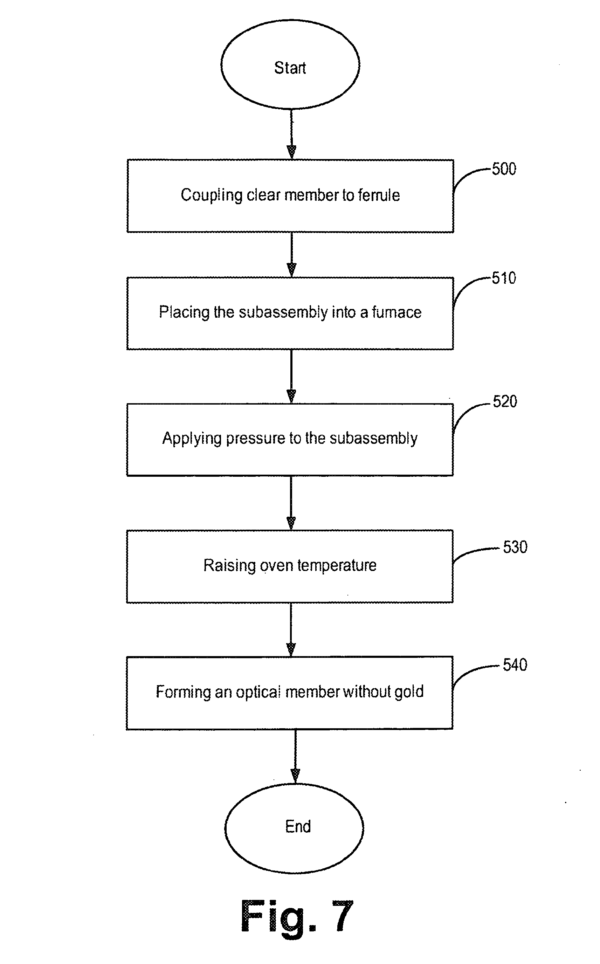

**FIG. 7** is a flow diagram that depicts one embodiment for forming an optical member either through an automatic or manual process. At block 500, a ferrule is coupled to a clear member to form a subassembly. Optionally, a surface of the clear window is metallized and the metallized surface is placed in direct contact with the ferrule. The metal is selected from a noble metal (except gold), a rare earth metal, Ti, Nb or other suitable materials. At block 520, the subassembly is placed into a furnace. At block 530, pressure is applied to the subassembly. At block 540, a temperature is raised in the furnace. At block 550, an optical member is formed without gold. An exemplary automatic process for forming an optical member includes a control module coupled to a "pick and place" device, and a metal introducer. A metal introducer device, optionally connected to the control module and/or the "pick and place" device, introduces one or more metals to the window and/or to the ferrule. The "pick and place" device is capable of picking up a ferrule and placing it into the setter plate. The "pick and place" device also is configured to "pick and place" the window and place into the setter plate. The "pick and place" device may continue this process with more than one setter plate, and then stack each plate to form a stacked assembly which is then placed into the oven. The control module starts and monitors the oven. Once the process is completed, the "pick and place" device removes the stacked assembly and then removes each finished optical member from each setter plate.

The present invention improves upon the traditional brazing method to join the ferrule to the clear member 204. For example, the optical feedthrough may be used with reduced or minimal polymeric overcoating, which is typically required for a gold brazed component in a body implant condition. Moreover, the batch process of diffusion bonding is able to process several thousand parts in a single run, compared to a much lower capacity for brazing in a traditional braze furnace. Diffusion bonding also substantially reduces the energy costs to form the optical member.

## Claims

1. A method of forming an optical member for an optical sensor in an implantable medical device comprising:
providing a ferrule (202);
coupling a clear window member (204) to the ferrule to form a subassembly;
placing the subassembly into a furnace;
applying pressure to the subassembly;
raising a temperature in the furnace; and
forming an optical member (200) without gold; wherein the ferrule and the clear window member are permanently attached to one another without joint material therebetween.

2. The method of any preceding claim wherein the pressure is applied in the range of 5-25 megaPascals (MPa).

3. The method of any preceding claim, wherein the temperature is 600-1500 Celsius (°C).

## Patentansprüche

1. Verfahren zum Bilden eines optischen Elements für einen optischen Sensor in einer implantierbaren medizinischen Vorrichtung, das umfasst:
Bereitstellen eines Ringbeschlags (202);
Koppeln eines durchsichtigen Fensterelements (204) mit dem Ringbeschlag, um eine Unteranordnung zu bilden;
Anordnen der Unteranordnung in einem Ofen;
Ausüben von Druck auf die Unteranordnung;
Erhöhen der Temperatur in dem Ofen; und
Bilden eines optischen Elements (200) ohne Gold;
wobei der Ringbeschlag und das durchsichtige Fensterelement ohne Verbindungsmaterial dazwischen dauerhaft aneinander befestigt werden.

2. Verfahren nach einem vorhergehenden Anspruch, wobei der Druck im Bereich von 5-25 Megapascal (MPa) ausgeübt wird.

3. Verfahren nach einem vorhergehenden Anspruch, wobei die Temperatur 600-1500 Grad Celsius (°C) beträgt.

## Revendications

1. Procédé de formation d'un élément optique pour un capteur optique dans un dispositif médical implantable, comportant les étapes consistant à :
fournir une ferrule (202) ;
coupler un élément de fenêtre transparent (204) à la ferrule pour former un sous-ensemble ;
placer le sous-ensemble dans un four ;
appliquer une pression au sous-ensemble ;
augmenter une température dans le four ; et
former un élément optique (200) sans or ; dans lequel la ferrule et l'élément de fenêtre transparent sont fixés de manière permanente l'un à l'autre sans matériau d'assemblage entre ceux-ci.

2. Procédé selon l'une quelconque des revendications précédentes, dans lequel la pression est appliquée dans la plage de 5 à 25 mégapascals (MPa).

3. Procédé selon l'une quelconque des revendications précédentes, dans lequel la température est de 600 à 1 500 degrés Celsius (°C).
